# FASCICULE DE BREVET EUROPEEN

(11) **EP 0 903 126 B1**
(45) Date de publication et mention de la délivrance du brevet: **10.08.2005**
(21) Numéro de dépôt: 98420154.1
(22) Date de dépôt: 10.09.1998
(51) Int. Cl.: A61F 2/38

(54) **Prothèse de genou à plateau rotatoire**
Knieprothese mit drehbarem Lagereinsatz
Knee prosthesis with rotating bearing

(30) Priorité: 23.09.1997 FR 9712042
(43) Date de publication de la demande: 24.03.1999
(60) Demande divisionnaire: 03015191.4
(73) Titulaire: Tornier SA, 38330 Saint Ismier (FR)
(72) Inventeur: Chambat, Pierre, 69003 LYON (FR); Judet, Thierry, 92410 Ville D'Avray (FR); Deschamps, Gérard, 71640 Givry (FR); Neyret, Philippe, 69300 Caluire (FR)
(74) Mandataire: Schmitt, John

(56) Documents cités:
- EP-A- 0 346 183
- EP-A- 0 634 156
- FR-A- 2 707 871
- FR-A- 2 735 017
- GB-A- 2 061 730
- GB-A- 2 312 377
- US-A- 4 728 332
- US-A- 5 282 868

## Description

La présente invention est relative à une prothèse de genou, et plus particulièrement à son dispositif d'assemblage du plateau tibial, réalisé en matière plastique, sur son embase métallique préalablement ancrée dans le tissu osseux tibial.

On connaît des prothèses de genou comprenant une partie tibiale en plastique qui est libre en rotation autour de l'axe osseux tibial par rapport à l'embase métallique solidaire du tibia.

Cette liberté de rotation est généralement réalisée par l'intermédiaire d'un axe mâle solidaire de la partie tibiale en matière plastique qui coopère avec un alésage ménagé dans l'embase métallique.

En outre, cette liberté de rotation peut être réalisée par un axe mâle solidaire de l'embase métallique qui coopère avec un alésage ménagé dans la partie tibiale en matière plastique.

Ces dispositifs de liaison comportent certains inconvénients, à savoir la difficulté d'insérer le plateau tibial en matière plastique une fois l'embase métallique en place sur le tibia. Ces inconvénients s'aggravent lors de la conservation du ligament croisé postérieur limitant l'accès à la partie supérieure du tibia.

De plus, on note que le centre de rotation est obligatoirement positionné à un endroit du dispositif ou l'alésage peut être réalisé, c'est à dire, dans une partie ou la matière est suffisante pour percer ledit alésage. Cette disposition du centre de rotation n'est pas forcément anatomique.

On constate que le volume engendré par l'axe osseux tibial peut empêcher la réalisation d'une échancrure postérieure sur le plateau tibial en matière plastique ou sur l'embase métallique pour la conservation du ligament croisé postérieur.

On connaît également des prothèses de genou comprenant un plateau tibial en matière plastique qui glisse librement sur la surface plane de l'embase métallique et dont le mouvement est limité par un ou deux ergots cylindriques solidaires de l'embase communiquant avec des espaces ménagés dans ledit plateau en matière plastique.

Ce type de prothèse présente l'inconvénient de ne pas matérialiser un axe de rotation.

EP-A-634 156 montre une prothèse selon le préambule de la revendication 1.

C'est à ces inconvénients qu'entend plus particulièrement remédier la présente invention.

La prothèse de genou suivant la présente invention à pour but de prévoir un plateau tibial en matière plastique présentant un degré de liberté en rotation par rapport à l'embase métallique.

La prothèse de genou conforme à la présente invention comporte une embase métallique et un plateau tibial qui sont pourvus de moyens de guidage définissant un centre de rotation (C,C') décalé par rapport à l'axe osseux tibial (YY'), de manière à permettre au plateau tibial de glisser en rotation sur ladite embase, lesdits moyens de guidage étant positionnés à une certaine distance du centre de rotation.

La prothèse de genou présente des moyens de guidage qui sont constitués dans la partie antérieure de l'embase métallique d'au moins une lèvre ou d'une série de plots en arc de cercle orienté dans une direction médio latérale et dans le plateau tibial par un logement destiné à coopérer avec l'élément de guidage de l'embase métallique pour permettre au plateau tibial de glisser en rotation autour du centre de rotation (C, C') de ladite lèvre ou plots.

La prothèse de genou suivant la présente invention comprend une embase métallique qui comporte une lèvre en arc de cercle présentant une partie centrale solidaire de bords latéraux de plus faible hauteur que celle prévue pour ladite partie centrale, tandis que le plateau tibial comprend sur sa face inférieure un logement en arc de cercle.

La prothèse de genou suivant la présente invention comprend une embase métallique qui comporte en face de la lèvre un plot de retenue porté par un centre de rotation afin de coopérer avec une échancrure ménagée dans le plateau tibial pour empêcher ce dernier de se soulever par rapport à l'embase lors du glissement en rotation dudit plateau autour de son centre de rotation.

La prothèse de genou suivant la présente invention comprend un plot de retenue qui est constitué d'un doigt cylindrique solidaire d'une tête de diamètre supérieur à celui prévu pour ledit doigt afin que ladite tête coopère avec des pans inclinés pratiqués dans l'échancrure.

La prothèse de genou suivant la présente invention comprend une embase métallique qui comporte en face de la lèvre un plot de centrage porté par le centre de rotation afin de coopérer avec un trou borgne ménagé dans le plateau tibial pour guider ce dernier par rapport à l'embase lors du glissement en rotation dudit plateau autour de son centre de rotation.

La prothèse de genou suivant la présente invention comprend une embase métallique et un plateau tibial qui comportent respectivement une échancrure pour le passage du ligament croisé postérieur.

La prothèse de genou suivant la présente invention comprend une embase métallique qui comporte deux lèvres en arc de cercle courbées dans le même sens et centrées autour du même centre de rotation, tandis que le plateau tibial comporte des logements destinés à recevoir respectivement lesdites lèvres pour permettre le glissement en rotation dudit plateau autour du centre de rotation.

La prothèse de genou suivant la présente invention comprend une lèvre qui est solidaire d'une collerette qui coopère dans une rainure du logement pour empêcher le plateau tibial de se soulever par rapport à l'embase métallique lors du glissement en rotation dudit plateau autour du centre de rotation.

La prothèse de genou suivant la présente invention comprend une embase métallique qui comporte deux lèvres en arc de cercle de sens inversés et centrées autour du même centre de rotation, tandis que le plateau tibial comporte un élément et un logement destinés à recevoir respectivement lesdites lèvres pour permettre le glissement en rotation dudit plateau autour du centre de rotation.

La prothèse de genou suivant la présente invention comprend une lèvre qui est disposée sur la périphérie externe du disque horizontal de l'embase métallique afin de coopérer avec un évidement périphérique du plateau tibial.

La prothèse de genou suivant la présente invention comprend une lèvre qui est décalée par rapport au centre de rotation et comportant une collerette qui vient s'encliqueter dans le logement du plateau tibial pour, d'une part guider lors du glissement en rotation le plateau autour de son centre, et d'autre part pour retenir ledit plateau afin que ce dernier ne se soulève pas de l'embase métallique.

La prothèse de genou suivant la présente invention comprend une embase métallique qui comporte une lèvre périphérique en arc de cercle solidaire d'une collerette dirigée en direction de l'axe vertical osseux du tibia et un logement disposé au niveau du centre de rotation, tandis que le plateau tibial présente sur sa périphérie externe un évidement dans lequel est ménagée une rainure horizontale destinée à recevoir la collerette de ladite lèvre et sur sa face inférieure un tenon qui coopère avec le logement.

La prothèse de genou suivant la présente invention comprend une embase métallique qui comporte trois lèvres périphériques s'étendant verticalement au-dessus du disque horizontal, tandis que le plateau tibial présente sur sa périphérie externe trois évidements destinés à recevoir respectivement lesdites lèvres pour permettre le guidage dudit plateau lors de ses glissements en rotation autour du centre de rotation.

La prothèse de genou suivant la présente invention comprend une embase métallique comportant au moins deux plots disposés en arc de cercle et définissant un centre de rotation (C, C'), tandis que le plateau tibial comporte un logement de même rayon de courbure ménagé dans le plateau tibial.

La prothèse de genou suivant la présente invention comprend des plots qui sont disposés en arc de cercle autour d'un centre de rotation (C, C'), tandis que le plateau tibial présente un logement destiné à recevoir lesdits plots.

La prothèse de genou suivant la présente invention comprend des plots qui présentent un centre de rotation (C') qui est décalé par rapport à l'axe vertical osseux du tibia (YY'), tandis que lesdits plots sont à une certaine distance de leur centre de rotation.

La prothèse de genou suivant la présente invention comprend un débattement en rotation entre le plateau tibial et l'embase métallique qui est réduit à zéro lorsque les dimensions du logement sont réalisées de manière à coopérer sans jeu avec la lèvre.

La prothèse de genou suivant la présente invention comprend une faible hauteur des moyens de guidage et de leurs positionnements antérieurs sur l'embase métallique qui autorise un montage du plateau tibial sur ladite embase par un abord strictement antérieur en ayant besoin de dégager ledit plateau vers le haut que de la hauteur desdits moyens de guidage.

La description qui va suivre au regard des dessins annexés, donnés à titre d'exemples non limitatifs, permettra de mieux comprendre l'invention, les caractéristiques qu'elle présente et les avantages qu'elle est susceptible de procurer :
Figure 1 est une vue en perspective éclatée, illustrant la prothèse de genou suivant la présente invention.
Figures 2 et 3 sont des vues de côté montrant la prothèse de genou avant la mise en place du plateau tibial en matière plastique sur l'embase métallique.
Figures 4 et 5 sont des vues représentant une première variante de la prothèse de genou suivant l'invention.
Figures 6, 7 et 8 sont supprimées.
Figures 9, 10 et 11 sont des vues montrant une seconde variante de la prothèse de genou dont l'embase métallique présente sur son axe de rotation un plot de retenue du plateau tibial en matière plastique.
Figures 12, 13 et 14 sont des vues montrant une troisième variante de la prothèse de genou dont l'embase métallique présente sur son axe de rotation un plot complémentaire de centrage du plateau tibial en matière plastique.
Figures 15, 16 et 17 sont des vues représentant une quatrième variante de la prothèse de genou qui est pourvue d'une échancrure pour le passage du ligament croisé postérieur.
Figures 18 à 24 sont des vues illustrant des variantes de la prothèse de genou dont l'embase métallique comporte deux lèvres de guidage opposées, mais dont l'une est portée par le centre de rotation de la seconde.
Figures 25 à 27 sont des vues représentant d'autres variantes de la prothèse de genou suivant la présente invention.
Figures 28 et 29 sont des vues illustrant des moyens de guidage disposés à la périphérie de l'embase métallique et du plateau tibial de la prothèse de genou.
Figures 30 à 32 sont des vues montrant des moyens de guidage constitués d'au moins deux plots verticaux solidaires de l'embase métallique qui coopèrent avec un logement ménagé dans le plateau tibial.

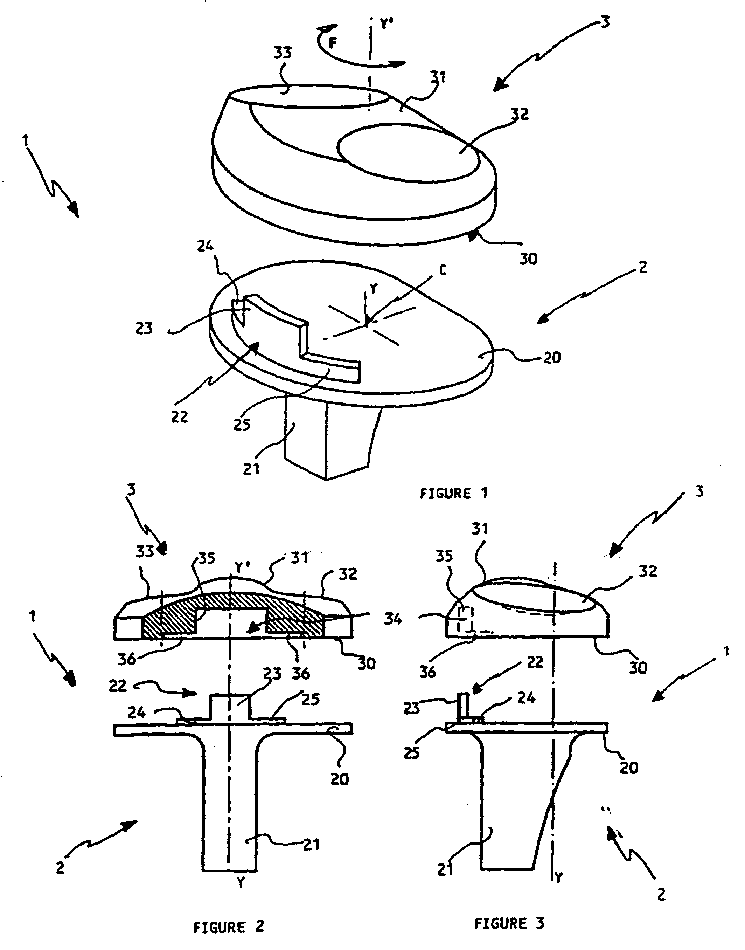

On a montré en figures 1 à 3 une prothèse de genou 1 comportant une embase métallique 2 et un plateau tibial 3, tandis que l'élément fémoral n'est pas représenté.

L'embase métallique 2 est constituée d'un disque horizontal 20 solidaire sur l'une de ses faces d'une tige d'ancrage 21 permettant la fixation de ladite embase dans le tibia d'un patient.

Le disque horizontal 20 comporte à l'opposé de la tige 21 des moyens de guidage qui sont constitués d'une lèvre 22 à profil extérieur en arc de cercle. Dans ce cas, on note que le centre de rotation C de la lèvre 22 est porté par l'axe vertical osseux du tibia YY'.

La lèvre 22, s'étendant verticalement au-dessus du disque horizontal 20, présente une partie centrale 23 solidaire de chaque côté de deux bords verticaux 24 et 25 de hauteur inférieure à celle prévue pour ladite partie centrale.

De plus, la lèvre 22 est positionnée sur le disque horizontal 20 de l'embase métallique 2 à une certaine distance du centre de rotation C.

Le plateau tibial 3, réalisé en matière plastique, présente une face inférieure plane 30, disposée dans un plan horizontal et parallèle à celui contenant le disque 20 de l'embase métallique.

A l'opposé de la face 30 le plateau tibial 3 comporte une face supérieure 31 munie de deux pistes 32 et 33 à profil concave, destinées à recevoir les condyles de l'élément fémoral non représenté.

La face inférieure 30 est percée d'un logement 34 de même rayon de courbure que celui prévu pour la lèvre 22 solidaire de l'embase métallique 2.

Le logement 34 présente des découpes 35 et 36 de plus grandes dimensions qui sont destinées à recevoir respectivement la partie centrale 23 et les bords latéraux 24 et 25 de la lèvre 22.

On constate lorsque le plateau tibial 3, et plus particulièrement sa face inférieure 30 en appuie sur le disque horizontal 20 de l'embase métallique 2, que la lèvre 22 pénètre à l'intérieur du logement 34.

Ce dernier présente des dimensions supérieures à celles prévues pour la lèvre 22 de manière que le plateau tibial 3 puisse glisser librement en rotation autour du centre de rotation C de ladite lèvre 22 suivant la flèche F illustrée en figure 1.

La lèvre 22 en arc de cercle ou incurvée est positionnée dans la partie antérieure de l'embase métallique 2 et orientée dans une direction sensiblement médio-latérale.

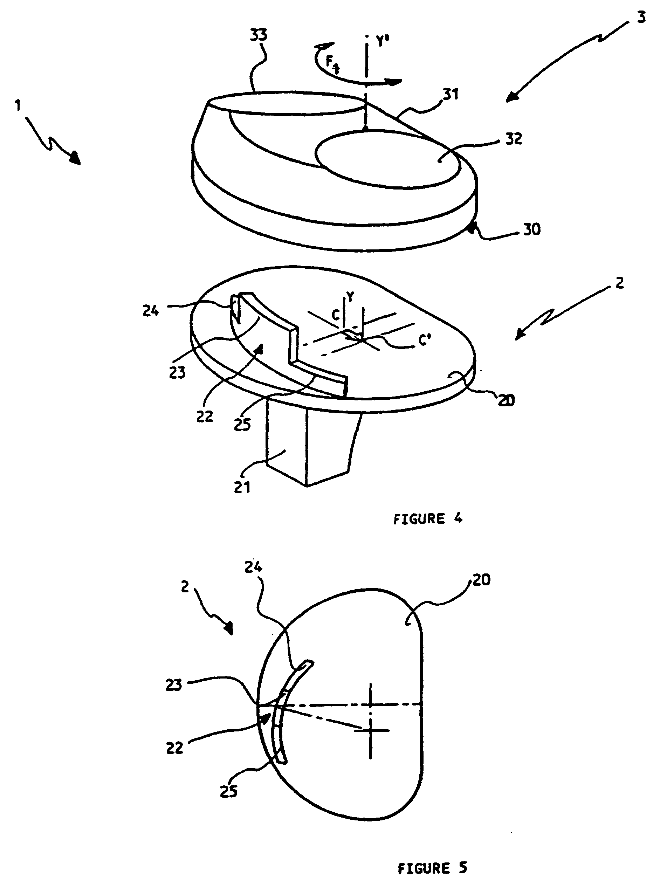

En figures 4 et 5 on a montré une première variante de la prothèse de genou 1 suivant la présente invention.

La prothèse de genou 1 est identique à celle décrite précédemment, c'est-à-dire qu'elle comprend une embase métallique 2 et un plateau tibial en matière plastique 3.

L'embase métallique 2 présente comme différence par rapport à celle ci-dessus, la position des moyens de guidage sur le disque horizontal 20. En effet, les moyens de guidage sont constitués d'une lèvre 22 en arc de cercle et dont le profil est semblable à celui décrit précédemment, mais dont le centre de rotation C' est décalé par rapport à celui C porté par l'axe vertical osseux du tibia YY'.

On note que le centre de rotation C' peut être positionné n'importe où, soit sur le disque horizontal 20, soit en dehors de celui-ci, tout en conservant les moyens de guidage sur ledit disque horizontal et plus particulièrement à un endroit.

De la même manière que précédemment, la lèvre 22 pourvue de sa partie centrale 23 et de ses bords latéraux 24 et 25, pénètre dans le logement 34 ménagé dans la face inférieure 30 du plateau tibial en matière plastique 3 pour assurer un glissement en rotation de ce dernier sur l'embase métallique 2 et autour du centre de rotation C' suivant la flèche F1 de la figure 4.

La lèvre 22 en arc de cercle ou incurvée est positionnée dans la partie antérieure de l'embase métallique 2 et orientée dans une direction sensiblement médio-latérale.

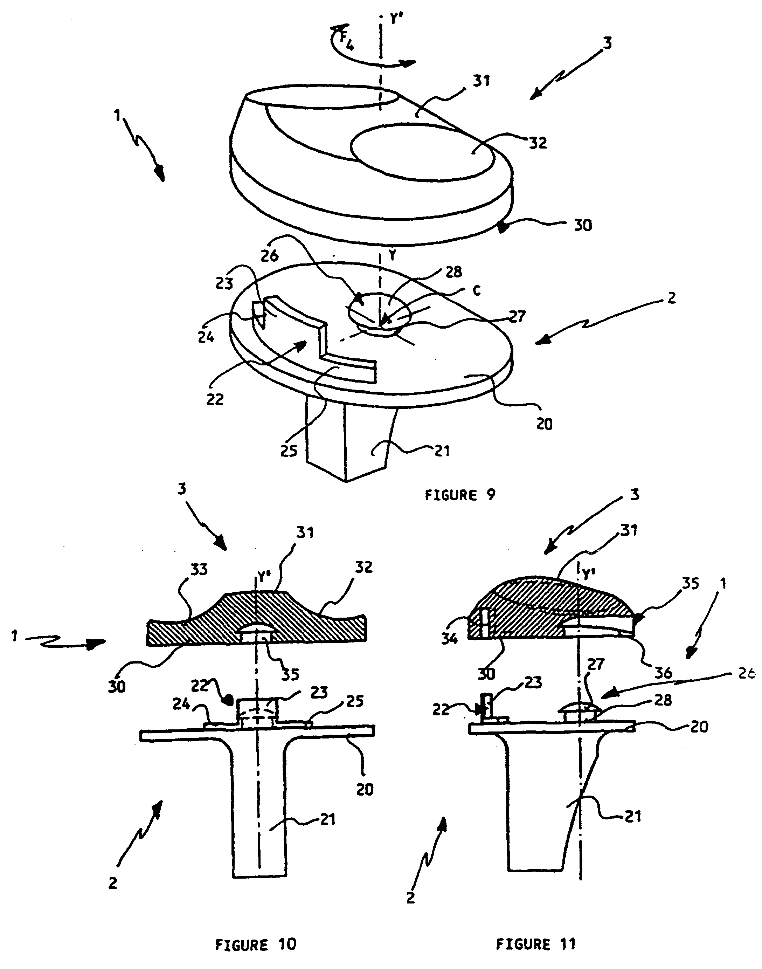

On a illustré en figures 9 à 11 une seconde variante de la prothèse de genou 1 suivant la présente invention.

La prothèse de genou 1 est identique à celle décrite en figures 1 à 3, à savoir qu'elle comprend une embase métallique 2 et un plateau tibial en matière plastique 3.

L'embase métallique 2 comporte sur son disque horizontal 20, et à l'opposé de sa tige d'ancrage 21, les moyens de guidage constitués par la lèvre 22 formée d'une partie centrale 23 et de deux bords latéraux 24 et 25.

En outre, le disque horizontal 20 comporte, au niveau du centre de rotation C de la lèvre 22 qui est portée par l'axe vertical osseux du tibia YY', un plot de retenue 26 s'étendant verticalement au-dessus dudit disque 20.

Le plot de retenue 26 constitue un moyen de guidage additionnel au moyen de guidage formé par la lèvre 22. Ainsi, le plot 26 est positionné sur le centre de rotation C du plateau tibial 3 sur l'embase métallique 2.

Le plot de retenue 26 est constitué d'un doigt cylindrique 27 solidaire d'une tête 28 dont le diamètre extérieur est supérieur à celui dudit doigt.

Le moyen de guidage additionnel ou plot 26 est solidaire du doigt 27, 28 pour empêcher le plateau tibial 3 de se soulever de l'embase métallique 2 lorsque la prothèse est en mouvement.

Le plateau tibial 3 présente sur sa face inférieure 30 le logement 34 recevant la lèvre 22 et une échancrure 35 à pans inclinés 36. Cette demière coopère lors de la mise en place du plateau 3 sur l'embase 2 avec le plot de retenue 26 de manière que la tête 28 soit disposée au-dessus des pans inclinés 36.

On constate que le plot de retenue 26, lorsqu'il coopère avec l'échancrure 35, permet d'empêcher, lors du glissement en rotation F4 du plateau tibial 3 sur l'embase métallique 2, que ledit plateau se soulève sous un effort de traction.

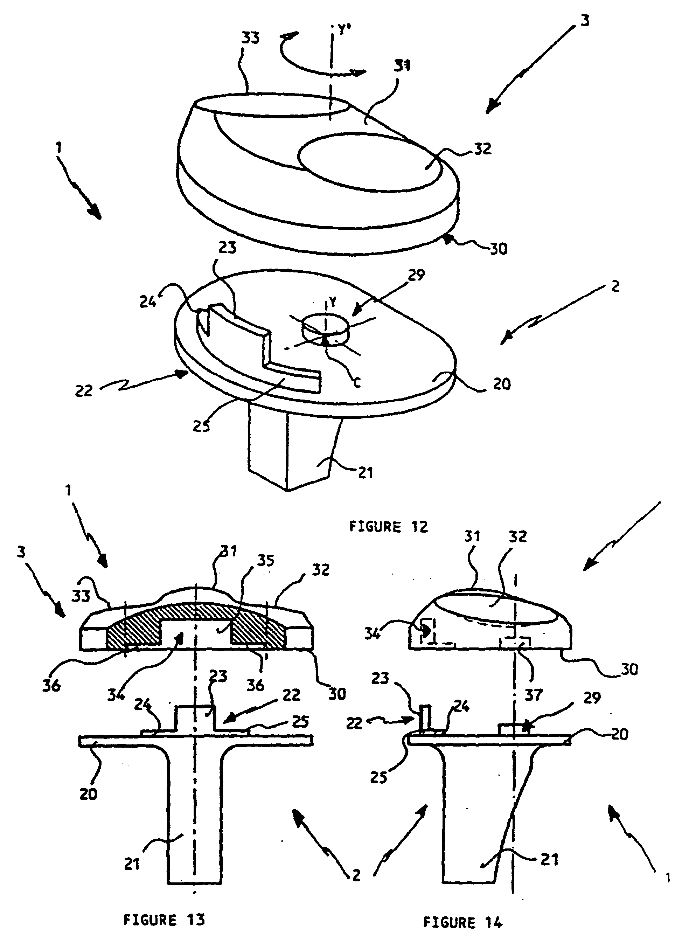

On a montré en figures 12 à 14 une troisième variante de la prothèse de genou 1 suivant l'invention.

Ainsi, l'embase métallique 2 comporte en face de la lèvre 22, et au niveau du centre de rotation C qui est porté par l'axe vertical osseux du tibia YY', un plot de centrage 29 s'étendant verticalement au-dessus du disque horizontal 20.

Le plot de centrage 29 s'étend verticalement au-dessus du disque horizontal 20 de faible hauteur, constituant un moyen de guidage additionnel au moyen de guidage formé par la lèvre 22.

Le plot de centrage 29 est constitué par un doigt cylindrique de faible hauteur.

Le plateau tibial en matière plastique 3 comprend sur sa face inférieure 30 et en face du logement 34 un trou borgne 37 destiné à recevoir le plot de centrage 29 lors de la mise en place dudit plateau sur l'embase métallique 2.

Le plot de centrage 29 permet de matérialiser le centre de rotation C de la lèvre 22 lors du glissement en rotation du plateau tibial 3 sur l'embase métallique 2.

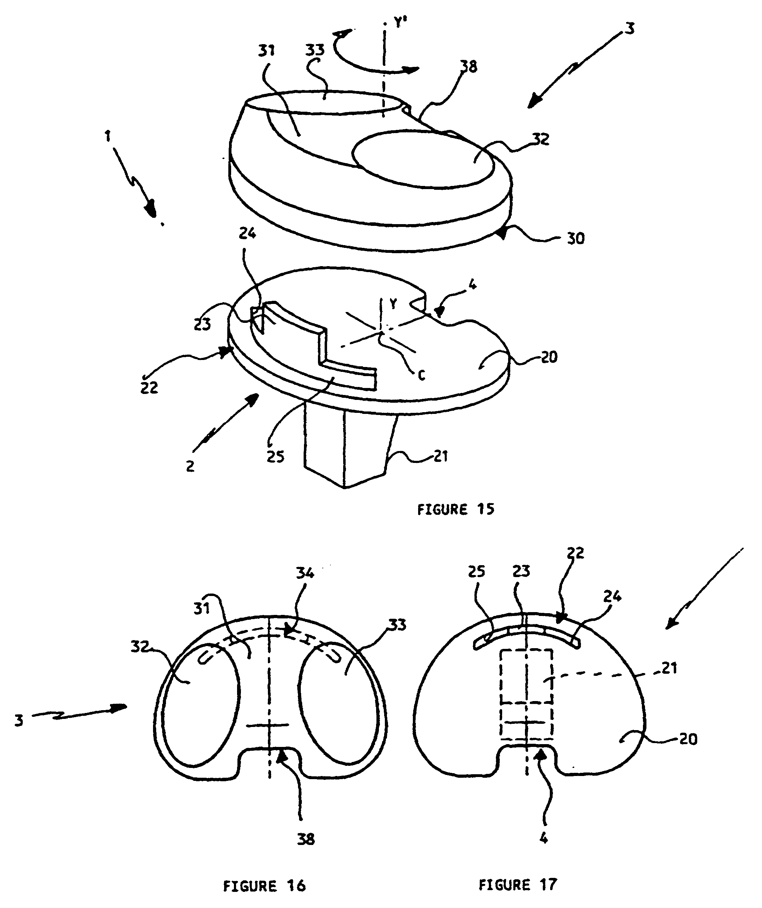

La prothèse de genou 1 illustrée en figures 15 à 17 diffère de celle montrée en figures 1 à 3 uniquement dans le fait que l'embase métallique 2 et que le plateau tibial en matière plastique 3 comportent respectivement une échancrure 4 et 38 pour le passage du ligament croisé postérieur.

Bien évidemment, la prothèse 1 montrée en figures 15 à 17 fonctionne de la même manière que celle décrite en figures 1 à 3.

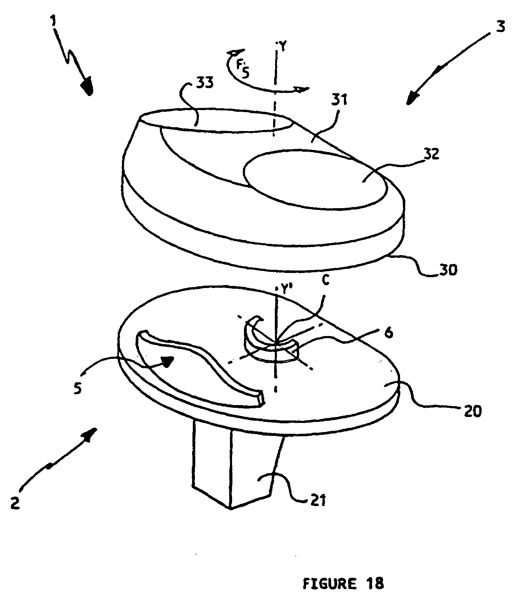

En figure 18 on a montré la prothèse de genou 1 munie de son embase métallique 2 et de son plateau tibial 3. L'embase métallique 2 comporte sur son disque horizontal 20 et plus particulièrement à l'opposé de la tige d'ancrage 21, une lèvre 5 en arc de cercle et de hauteur variable. Cette dernière est prévue d'un profil différent de ceux constituant les lèvres décrites précédemment. Au niveau du centre de rotation C de la lèvre 5 est prévu une seconde lèvre 6 en arc de cercle.

Les lèvres 5 et 6 coopèrent dans des logements non représentés, mais ménagés sur la face 30 du plateau tibial 3 pour permettre le guidage suivant un glissement en rotation dudit plateau autour du centre de rotation C, comme cela est matérialisé par la flèche F5.

La lèvre 5 en arc de cercle ou incurvée est positionnée dans la partie antérieure de l'embase métallique 2 et orientée dans une direction sensiblement médio-latérale.

La lèvre 6 constitue un moyen de guidage additionnel au moyen de guidage formé par la lèvre 5. Ainsi, la lèvre 6 est positionnée sur le centre de rotation C du plateau tibial 3 sur l'embase métallique 2.

On peut prévoir que le centre de rotation des lèvres 5 et 6 soit décalé de l'axe vertical osseux du tibia YY' sans pour autant changer l'objet de l'invention.

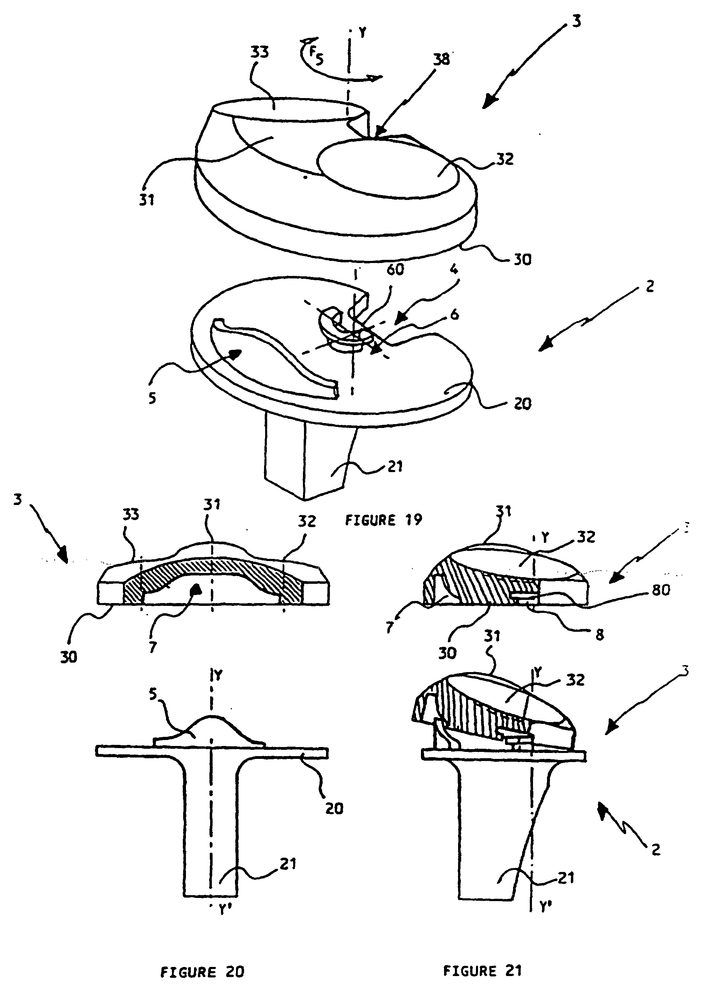

En figures 19 à 21 on a représenté une variante de la prothèse de genou 1 montrée en figure 18, à savoir que la lèvre 6 est solidaire d'une collerette 60 formant une sorte de petit plateau disposé dans un plan horizontal parallèle à celui contenant le disque 20 de l'embase métallique 2.

Le plateau tibial 3 présente sur sa face 30 des logements 7 et 8 destinés à recevoir respectivement les lèvres 5 et 6 pour permettre un glissement en rotation du plateau tibial 3 sur l'embase métallique 2 autour du centre de rotation C et suivant la flèche F5.

Le logement 7 présente un profil sensiblement identique à celui de la lèvre 5 et, tout du moins, en arc de cercle pour guider, lors de ses déplacements, le plateau tibial 3.

Le logement 8 présente un profil sensiblement identique à la collerette 60 du moyen de guidage additionnel ou lèvre 6 pour guider, lors de ses déplacements, le plateau tibial 3.

Le logement 8 est percé d'une rainure interne 80 destinée à recevoir la collerette 60 de la lèvre 6 pour effectuer une sorte d'encliquetage du plateau tibial 3 sur l'embase 2, afin que ledit plateau ne puisse se soulever lors d'un effort de traction.

On note que les lèvres 5 et 6 en arc de cercle sont courbées dans le même sens et suivant le même centre de rotation C ou C', lorsque ce dernier est décalé de l'axe vertical osseux du tibia YY'.

Comme en figure 15 l'embase métallique 2 et le plateau tibial 3 peuvent comprendre respectivement les échancrures 4 et 38 pour le passage du ligament croisé postérieur.

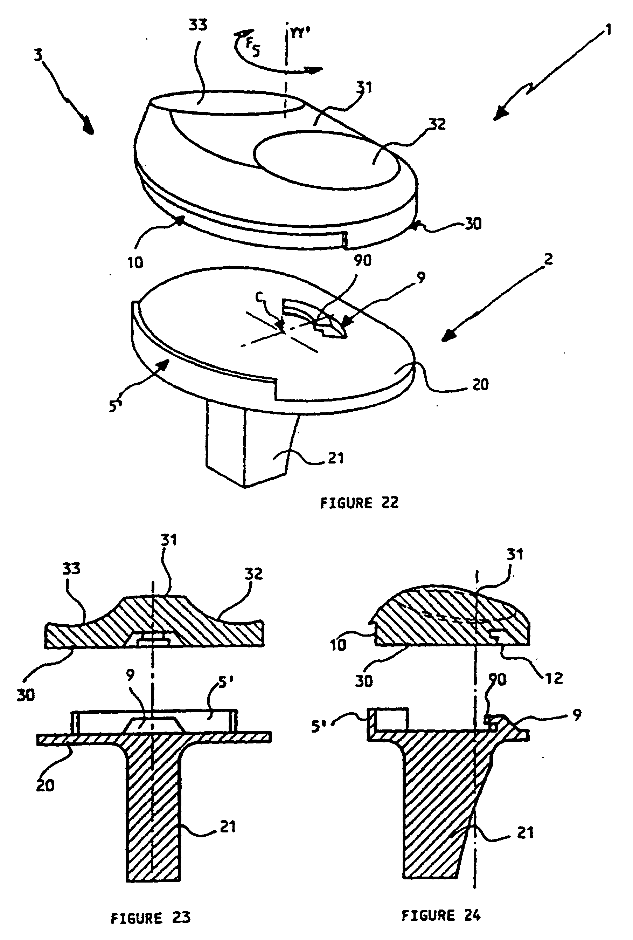

En figures 22 à 24 la prothèse de genou 1 comporte sur son embase métallique 2 une première lèvre 5' disposée à la périphérie externe du disque horizontal 20. En face de la lèvre 5' le disque horizontal 20 est solidaire d'une autre lèvre 9 en arc de cercle, mais dont le rayon de courbure est inversé par rapport à celui prévu pour la lèvre 5'.

La lèvre 5 en arc de cercle ou incurvée est positionnée dans la partie antérieure de l'embase métallique 2 et orientée dans une direction sensiblement médio-latérale.

La lèvre 9 constitue un moyen de guidage additionnel au moyen de guidage formé par la lèvre 5'. Ainsi, la lèvre 9 est positionnée sur le centre de rotation C du plateau tibial 3 sur l'embase métallique 2.

La lèvre 9 présente le même centre de rotation C que celui de la lèvre 5', mais ledit centre peut être décalé, suivant la configuration de la prothèse de genou, de l'axe vertical osseux du tibia YY'.

La lèvre 9 est solidaire d'une collerette 90 dont on verra mieux plus loin la fonction.

Le plateau tibial 3 présente sur sa périphérie inférieure, c'est-à-dire celle comprise entre les faces 30 et 31, un évidement 10 recevant la lèvre 5' lors de la mise en place du plateau tibial 3 sur l'embase métallique 2.

A l'opposé de l'évidement 10, la face inférieure 30 est percée d'un logement 12 dans lequel vient s'encliqueter la lèvre 9 pour, d'une part guider lors du glissement en rotation le plateau 3 autour de son centre C, et d'autre part retenir ledit plateau pour éviter que ce dernier ne se soulève de l'embase métallique 2.

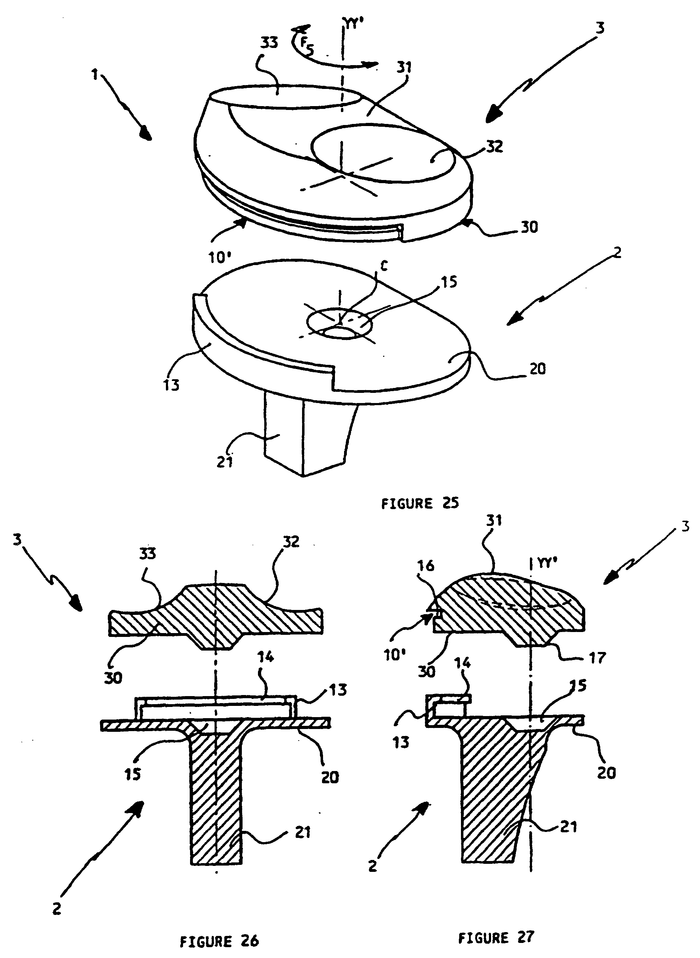

En figures 25 à 27 la prothèse de genou 1 comporte l'embase métallique 2 dont le disque horizontal 20 présente sur sa périphérie externe une lèvre 13 en arc de cercle solidaire d'une collerette 14 dirigée en direction de l'axe vertical osseux du tibia YY'.

Le disque horizontal 20 est percé au niveau du centre de rotation C de la lèvre 13 d'un logement 15 en forme de cuvette constituant un moyen de guidage additionnel au moyen de guidage formé par ladite lèvre 13. Cette demière en arc de cercle ou incurvée est positionnée dans la partie antérieure de l'embase métallique 2 et orientée dans une direction sensiblement médio-latérale.

La lèvre 13 et le logement 15 peuvent comprendre un centre de rotation C qui est décalé de l'axe vertical osseux du tibia YY'.

Le plateau tibial 3 présente sur sa périphérie externe et entre les faces 30 et 31, un évidement 10' dans lequel est ménagée une rainure horizontale 16 destinée à recevoir la collerette 14 de la lèvre 13 lors du glissement en rotation du plateau 3 sur l'embase métallique 2.

La face inférieure 30 est solidaire d'un tenon 17 à profil conique apte à coopérer avec le logement 15 ménagé dans le disque horizontal 20 de l'embase métallique 2.

La lèvre 13 munie de sa collerette 14, le logement 15, l'évidement 10' et sa rainure 16, et le tenon 17 constituent les moyens de guidage du plateau tibial 3 sur l'embase métallique 2 lors du glissement en rotation dudit plateau suivant la flèche F5.

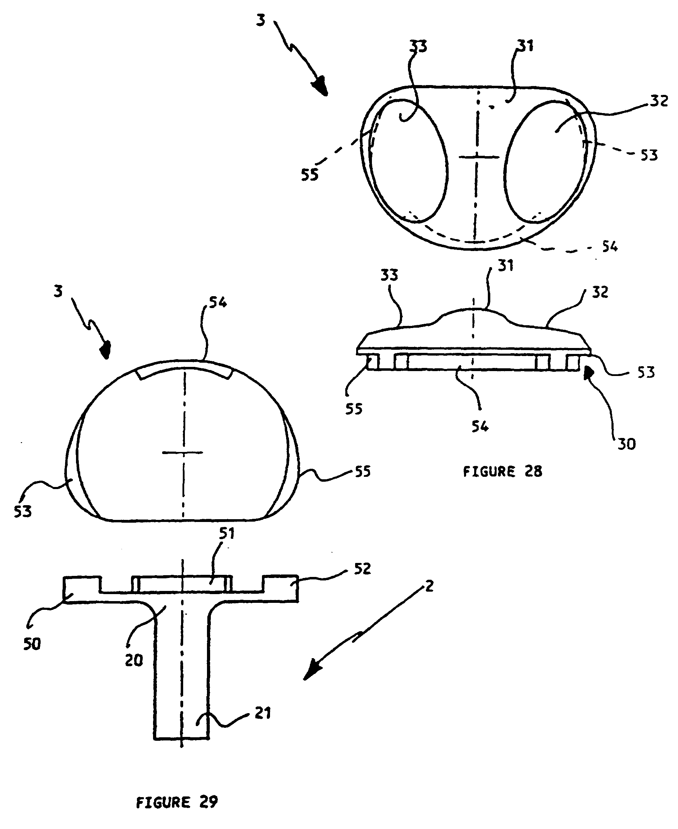

En figures 28 et 29 l'embase métallique 2 de la prothèse de genou 1 comporte sur son disque horizontal 20 trois lèvres périphériques 50, 51 et 52 s'étendant verticalement au-dessus dudit disque horizontal 20.

Le plateau tibial 3 comporte sur sa périphérie externe trois évidements périphériques 53, 54 et 55 destinés à recevoir respectivement les lèvres 50, 51 et 52 pour permettre le guidage dudit plateau lors de ses glissements en rotation autour du centre de rotation desdites lèvres qui est identique pour les trois.

La lèvre 51 en arc de cercle ou incurvée est positionnée dans la partie antérieure de l'embase métallique 2 et orientée dans une direction sensiblement médio-latérale.

Les lèvres 50, 52 en arc de cercle constituent des moyens de guidage additionnels au moyens de guidage formés par la lèvre 51. Les lèvres 50, 52 sont positionnées à proximité du centre de rotation C du plateau tibial 3 sur l'embase métallique 2.

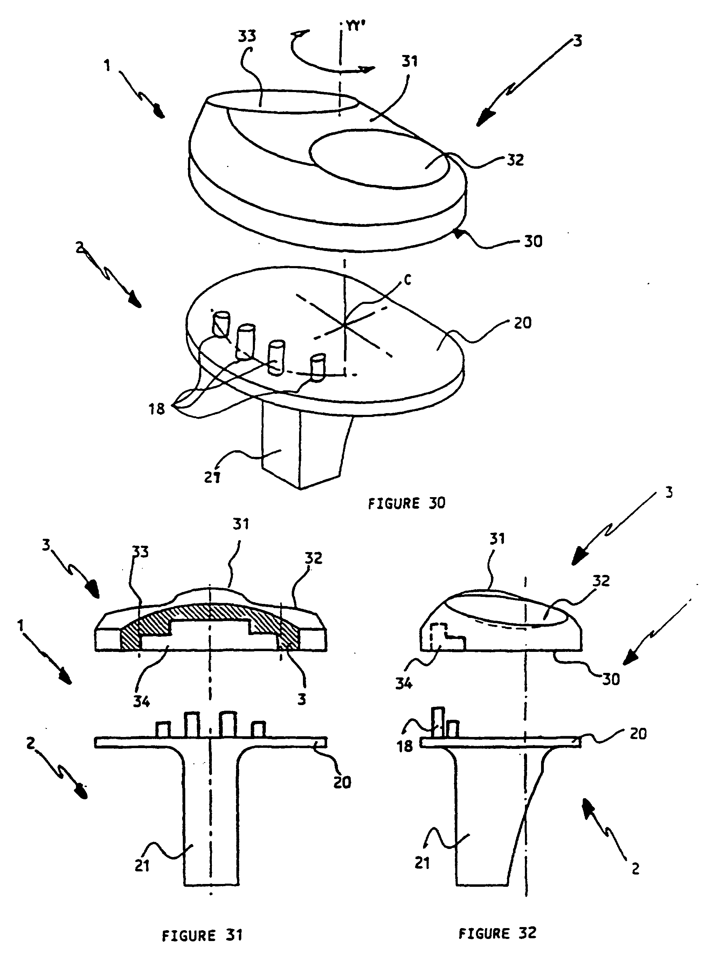

En figures 30 à 32 l'embase métallique 2 de la prothèse de genou 1 comporte sur son disque horizontal 20 des plots verticaux 18 disposés en arc de cercle autour d'un centre de rotation C qui peut être décalé de l'axe vertical osseux du tibia YY'.

Les plots 18 en arc de cercle sont positionnés dans la partie antérieure de l'embase métallique 2 et orientés dans une direction sensiblement médio-latérale.

Dans cet exemple, le plateau tibial 3 est identique à celui décrit en figure 1 à 3, c'est-à-dire que sa face inférieure 30 comporte un logement 34 destiné à recevoir les plots 18 pour le guidage lors du glissement en rotation dudit plateau par rapport à l'embase métallique 2.

On constate que dans chaque exemple décrit ci-dessus, il est possible, suivant les cas, de décaler ou non le centre de rotation des lèvres ou des plots pour déterminer un glissement en rotation particulier du plateau tibial 3 par rapport à l'embase métallique 2.

On note également que dans chaque exemple décrit ci-dessus, il peut être prévu ou non des échancrures 4 et 38 pour le passage du ligament croisé postérieur.

En outre, on remarque que les moyens de guidage de chaque exemple décrit ci-dessus sont positionnés à une certaine distance du centre de rotation C, C'.

On remarque que la faible hauteur de la lèvre ou des plots, et sa position antérieure sur l'embase métallique 2, permet la mise en place aisée du plateau en matière plastique 3 sur ladite embase, par un abord strictement antérieur, en ayant besoin de dégager le plateau vers le haut, que de la hauteur de ladite lèvre ou desdits plots comme montrés en figure 21.

On constate par ailleurs que le plateau en matière plastique 3 ne peut glisser en rotation sur l'embase métallique 2 que dans les limites fixées par la différence de dimensions entre le logement et la lèvre ou plot correspondant, ce qui permet d'empêcher tout excès de mouvements non souhaités.

Dans ces conditions, on comprend aisément que l'on peut obtenir un plateau en matière plastique 3 dont les dimensions du logement sont identiques à celles de la lèvre ou des plots correspondants pour empêcher tout débattement dudit plateau sur l'embase métallique 2. Ceci permet au chirurgien, suivant les cas opératoires particuliers, de revenir à un système de prothèse de genou à plateau en matière plastique qui est fixé sur l'embase métallique 2 sans avoir besoin de changer cette dernière.

## Revendications

1. Prothèse de genou comportant une embase métallique (2) solidaire d'une tige d'ancrage pour sa fixation dans le tibia d'un patient, un plateau tibial (3) en matière plastique et des moyens de guidage permettant au plateau tibial de pouvoir glisser librement sur ladite embase autour d'un centre de rotation (C,C') décalé par rapport à l'axe osseux tibial (YY'), **caractérisée en ce que** les moyens de guidage sont constitués dans la partie antérieure de l'embase métallique (2) d'au moins une lèvre ou d'une série de plots (22, 5, 5',13, 51, 18) en arc de cercle orienté dans une direction médio latérale et dans le plateau tibial (3) par un logement (34, 7, 10, 10', 54) destiné à coopérer avec l'élément de guidage de l'embase métallique (2) pour permettre au plateau tibial (3) de glisser en rotation autour du centre de rotation (C, C') de ladite lèvre ou plot.

2. Prothèse de genou suivant la revendication 1, **caractérisée en ce que** l'embase métallique (2) comporte une lèvre (22) en arc de cercle présentant une partie centrale (23) solidaire de bords latéraux (24, 25) de plus faible hauteur que celle prévue pour ladite partie centrale, tandis que le plateau tibial (3) comprend sur sa face inférieure (30) un logement (34) en arc de cercle.

3. Prothèse de genou suivant la revendication 2, **caractérisée en ce que** l'embase métallique (2) comporte en face de la lèvre (22) un plot de retenue (26) porté par un centre de rotation afin de coopérer avec une échancrure (35) ménagée dans le plateau tibial (3) pour empêcher ce dernier de se soulever par rapport à l'embase (2) lors du glissement en rotation dudit plateau autour de son centre de rotation.

4. Prothèse de genou suivant la revendication 3, **caractérisée en ce que** le plot de retenue (26) est constitué d'un doigt cylindrique (27) solidaire d'une tête (28) de diamètre supérieur à celui prévu pour ledit doigt afin que ladite tête coopère avec des pans inclinés pratiqués dans l'échancrure (35).

5. Prothèse de genou suivant la revendication 2, **caractérisée en ce que** l'embase métallique (2) comporte en face de la lèvre (22) un plot de centrage (29) porté par le centre de rotation (C, C') afin de coopérer avec un trou borgne (37) ménagé dans le plateau tibial (3) pour guider ce dernier par rapport à l'embase (2) lors du glissement en rotation dudit plateau autour de son centre de rotation.

6. Prothèse de genou suivant la revendication 1, **caractérisée en ce que** l'embase métallique (2) et le plateau tibial (3) comportent respectivement une échancrure (4 et 38) pour le passage du ligament croisé postérieur.

7. Prothèse de genou suivant la revendication 1, **caractérisée en ce que** l'embase métallique (2) comporte deux lèvres (5 et 6) en arc de cercle courbées dans le même sens et centrées autour du même centre de rotation (C, C'), tandis que le plateau tibial (3) comporte des logements (7 et 8) destinés à recevoir respectivement lesdites lèvres (5 et 6) pour permettre le glissement en rotation dudit plateau autour du centre de rotation (C, C').

8. Prothèse de genou suivant la revendication 7, **caractérisée en ce que** la lèvre (6) est solidaire d'une collerette (60) qui coopère dans une rainure (80) du logement (8) pour empêcher le plateau tibial (3) de se soulever par rapport à l'embase métallique (2) lors du glissement en rotation dudit plateau autour du centre de rotation (C, C').

9. Prothèse de genou suivant la revendication 1, **caractérisée en ce que** l'embase métallique (2) comporte deux lèvres (5' et 9) en arc de cercle de sens inversés et centrées autour du même centre de rotation (C, C'), tandis que le plateau tibial (3) comporte un élément (10) et un logement (12) destinés à recevoir respectivement lesdites lèvres (5' et 9) pour permettre le glissement en rotation dudit plateau autour du centre de rotation (C, C').

10. Prothèse de genou suivant la revendication 9, **caractérisée en ce que** la lèvre (5') est disposée sur la périphérie externe du disque horizontal (20) de l'embase métallique (2) afin de coopérer avec un évidement périphérique (10) du plateau tibial (3).

11. Prothèse de genou suivant la revendication 9, **caractérisée en ce que** la lèvre (9) est décalée par rapport au centre de rotation (C, C') et comporte une collerette (90) qui vient s'encliqueter dans le logement (12) du plateau tibial (3) pour, d'une part guider lors du glissement en rotation le plateau (3) autour de son centre (C, C'), et d'autre part pour retenir ledit plateau afin que ce dernier ne se soulève pas de l'embase métallique (2).

12. Prothèse de genou suivant la revendication 1, **caractérisée en ce que** l'embase métallique (2) comporte une lèvre périphérique (13) en arc de cercle solidaire d'une collerette (14) dirigée en direction de l'axe vertical osseux du tibia (YY') et un logement (15) disposé au niveau du centre de rotation (C, C'), tandis que le plateau tibial (3) présente sur sa périphérie externe un évidement (10') dans lequel est ménagée une rainure horizontale (16) destinée à recevoir la collerette (14) de ladite lèvre (13) et sur sa face inférieure (30) un tenon (17) qui coopère avec le logement (15).

13. Prothèse de genou suivant la revendication 1, **caractérisée en ce que** l'embase métallique (2) comporte trois lèvres périphériques (50, 51, 52) s'étendant verticalement au-dessus du disque horizontal (20), tandis que le plateau tibial (3) présente sur sa périphérie externe trois évidements (53, 54, 55) destinés à recevoir respectivement lesdites lèvres (50, 51, 52) pour permettre le guidage dudit plateau lors de ses glissements en rotation autour du centre de rotation (C, C').

14. Prothèse de genou suivant la revendication 1, **caractérisé en ce que** l'embase métallique (2) comporte au moins deux plots (18) disposés en arc de cercle et définissant un centre de rotation (C, C'), tandis que le plateau tibial (3) comporte un logement (34) de même rayon de courbure ménagé dans le plateau tibial (3).

15. Prothèse de genou suivant la revendication 14, **caractérisé en ce que** les plots (18) sont disposés en arc de cercle autour d'un centre de rotation (C, C'), tandis que le plateau tibial (3) présente un logement (34) destiné à recevoir lesdits plots.

16. Prothèse de genou suivant la revendication 14, **caractérisé en ce que** les plots (18) présentent un centre de rotation (C') qui est décalé par rapport à l'axe vertical osseux du tibia (YY'), tandis que lesdits plots sont à une certaine distance de leur centre de rotation.

17. Prothèse de genou suivant la revendication 1, **caractérisée en ce que** le débattement en rotation entre le plateau tibial (3) et l'embase métallique (2) est réduit à zéro lorsque les dimensions du logement (34, 7, 10, 10', 54) sont réalisées de manière à coopérer sans jeu avec la lèvre (22, 5, 5', 13, 51).

18. Prothèse de genou suivant la revendication 1, **caractérisée en ce que** la faible hauteur des moyens de guidage et de leurs positionnements antérieurs sur l'embase métallique (2) autorise un montage du plateau tibial (3) sur ladite embase par un abord strictement antérieur en ayant besoin de dégager ledit plateau vers le haut que de la hauteur desdits moyens de guidage.

## Patentansprüche

1. Knieprothese mit einem Metallsockel (2), der mit einem Verankerungsschaft zu seiner Befestigung im Schienbein eines Patienten fest verbunden ist, einer Schienbeinplatte (3) aus Kunststoff und Mitteln zum Führen, die es der Schienbeinplatte erlauben, frei auf dem Sockel um einen Drehpunkt (C, C') in Bezug auf die Schienbeinknochenachse (YY') versetzt zu gleiten, **dadurch gekennzeichnet, dass** die Führungsmittel im vorderen Teil des Metallsockels (2) aus mindestens einer Kreisbogenlippe oder mehrere Kreisbogenklötze (22, 5, 5', 13, 51, 18) in eine mediolaterale Richtung gerichtet bestehen und in der Schienbeinplatte (3) aus einer Aufnahme (34, 7, 10, 10', 54), die dazu bestimmt ist, mit dem Führungselement des Metallsockels (2) zusammenzuwirken, um es der Schienbeinplatte (3) zu erlauben, in Drehung um den Drehpunkt (C, C') der Lippe oder des Klotzes zu gleiten.

2. Knieprothese nach Anspruch 1, **dadurch gekennzeichnet, dass** der Metallsockel (2) eine Kreisbogenlippe (22) umfasst, die einen mittleren Teil (23) aufweist, der fest mit den seitlichen Rändern (24, 25) mit geringerer Höhe als der für den mittleren Teil vorgesehenen verbunden ist, während die Schienbeinplatte (3) auf ihrer unteren Fläche (30) eine Kreisbogenaufnahme (34) aufweist.

3. Knieprothese nach Anspruch 2, **dadurch gekennzeichnet, dass** der Metallsockel (2) gegenüber der Lippe (22) einen Rückhalteklotz (26) umfasst, der von einem Drehpunkt getragen wird, um mit einem Ausschnitt (35) zusammenzuwirken, der in der Schienbeinplatte (3) eingerichtet ist, um letztere daran zu hindern, sich in Bezug auf den Sockel (2) beim Gleiten in Drehung der Platte um ihren Drehpunkt abzuheben.

4. Knieprothese nach Anspruch 3, **dadurch gekennzeichnet, dass** der Rückhalteklotz (26) aus einem zylindrischen Finger (27) besteht, der fest mit einem Kopf (28) mit einem Durchmesser verbunden ist, der größer ist als der für den Finger vorgesehene, damit der Kopf mit den schrägen Flächen, die im Ausschnitt (35) angebracht sind, zusammenwirkt.

5. Knieprothese nach Anspruch 2, **dadurch gekennzeichnet, dass** der Metallsockel (2) gegenüber der Lippe (22) einen Zentrierklotz (29) umfasst, der vom Drehpunkt (C, C') getragen wird, um mit einem Sackloch (37) zusammenzuwirken, das in der Schienbeinplatte (3) eingerichtet ist, um letztere in Bezug auf den Sockel (2) beim Gleiten in Drehung der Platte um ihren Drehpunkt zu führen.

6. Knieprothese nach Anspruch 1, **dadurch gekennzeichnet, dass** der Metallsockel (2) und die Schienbeinplatte (3) jeweils einen Ausschnitt (4 und 38) für die Passage des hinteren Kreuzbands umfasst.

7. Knieprothese nach Anspruch 1, **dadurch gekennzeichnet, dass** der Metallsockel (2) zwei Kreisbogenlippen (5 und 6) in die gleiche Richtung gekrümmt und um den gleichen Drehpunkt (C, C') zentriert umfasst, während die Schienbeinplatte (3) Aufnahmen (7 und 8) umfasst, die dazu bestimmt sind, jeweils die Lippen (5 und 6) aufzunehmen, um das Gleiten in Drehung der Platte um den Drehpunkt (C, C') zu erlauben.

8. Knieprothese nach Anspruch 7, **dadurch gekennzeichnet, dass** die Lippe (6) fest mit einem Kragen (60) verbunden ist, der in einer Rille (80) der Aufnahme (8) zusammenwirkt, um die Schienbeinplatte (3) daran zu hindern, sich in Bezug auf den Metallsockel (2) beim Gleiten in Drehung der Platte um den Drehpunkt (C, C') anzuheben.

9. Knieprothese nach Anspruch 1, **dadurch gekennzeichnet, dass** der Metallsockel (2) auch zwei Kreisbogenlippen (5' und 9) in umgekehrte Richtungen und um den gleichen Drehpunkt (C, C') zentriert umfasst, während die Schienbeinplatte (3) ein Element (10) und eine Aufnahme (12) umfasst, die dazu bestimmt sind, jeweils die Lippen (5' und 9) aufzunehmen, um das Gleiten in Drehung der Platte um den Drehpunkt (C, C') zu erlauben.

10. Knieprothese nach Anspruch 9, **dadurch gekennzeichnet, dass** die Lippe (5') am externen Rand der horizontalen Scheibe (20) des Metallsockels (2) angeordnet ist, um mit einer peripheren Aussparung (10) der Schienbeinplatte (3) zusammenzuwirken.

11. Knieprothese nach Anspruch 9, **dadurch gekennzeichnet, dass** die Lippe (9) in Bezug auf den Drehpunkt (C, C') versetzt ist und einen Kragen (90) umfasst, der in die Aufnahme (12) der Schienbeinplatte (3) einrastet, um einerseits die Platte (3) beim Gleiten in Drehung um ihren Drehpunkt (C, C') zu führen und sie andererseits zurückzuhalten, damit sie sich nicht vom Metallsockel (2) abhebt.

12. Knieprothese nach Anspruch 1, **dadurch gekennzeichnet, dass** der Metallsockel (2) eine periphere Kreisbogenlippe (13), die fest mit einem Kragen (14) verbunden ist, der in die Richtung der senkrechten Knochenachse des Schienbeins (YY') gerichtet ist, und eine Aufnahme (15) umfasst, die auf der Ebene des Drehpunkts (C, C') angeordnet ist, während die Schienbeinplatte (3) an ihrer externen Peripherie eine Aussparung (10') aufweist, in der eine horizontale Rille (16) eingerichtet ist, die dazu bestimmt ist, den Kragen (14) der Lippe (13) aufzunehmen und auf seiner unteren Seite (30) einen Zapfen (17), der mit der Aufnahme (15) zusammenwirkt.

13. Knieprothese nach Anspruch 1, **dadurch gekennzeichnet, dass** der Metallsockel (2) drei periphere Lippen (50, 51, 52) umfasst, die sich senkrecht über der horizontalen Scheibe (20) erstrecken, während die Schienbeinplatte (3) auf ihrer externen Peripherie drei Aussparungen (53, 54, 55) umfasst, die dazu bestimmt sind, jeweils die Lippen (50, 51, 52) aufzunehmen, um das Führen der Platte bei ihren Gleitbewegungen in Drehung um den Drehpunkt (C, C') zu erlauben.

14. Knieprothese nach Anspruch 1, **dadurch gekennzeichnet, dass** der Metallsockel (2) mindestens zwei Klötze (18) umfasst, die im Kreisbogen angeordnet sind und einen Drehpunkt (C, C') definieren, während die Schienbeinplatte (3) eine Aufnahme (34) mit dem gleichen Krümmungsradius umfasst.

15. Knieprothese nach Anspruch 14, **dadurch gekennzeichnet, dass** die Klötze (18) im Kreisbogen um einen Drehpunkt (C, C') angeordnet sind, während die Schienbeinplatte (3) eine Aufnahme (34) umfasst, die zur Aufnahme der Klötze bestimmt ist.

16. Knieprothese nach Anspruch 14, **dadurch gekennzeichnet, dass** die Klötze (18) einen Drehpunkt (C') aufweisen, der in Bezug auf die senkrechte Knochenachse des Schienbeins (YY') versetzt ist, während sich der Klotz in einer bestimmten Entfernung von seinem Drehpunkt befindet.

17. Knieprothese nach Anspruch 1, **dadurch gekennzeichnet, dass** das Schwenken in Drehung zwischen der Schienbeinplatte (3) und dem Metallsockel (2) auf Null reduziert ist, wenn die Maße der Aufnahme (34, 7, 10, 10', 54) so hergestellt werden, dass sie ohne Spiel mit der Lippe (22, 5, 5', 13, 51) zusammenwirken.

18. Knieprothese nach Anspruch 1, **dadurch gekennzeichnet, dass** die geringe Höhe der Führungsmittel und ihre vorderen Positionierungen auf dem Metallsockel (2) eine Installation der Schienbeinplatte (3) auf dem Sockel über einen strikt vorderen Rand erlauben, wobei die Platte nur um die Höhe der Führungsmittel nach oben freigefahren wird.

## Claims

1. Knee prosthesis comprising a metal base (2) secured to an anchoring rod for fixing it into the tibia of a patient, a plastic tibia plate (3) and guide means allowing the tibia plate to slide freely over the said base about a centre of rotation (C, C') offset from the tibia bone axis (YY'), **characterized in that** the guide means consist, in the anterior part of the metal base (2), of at least one upstand or a series of pegs (22, 5, 5', 13, 51, 18) in the shape of an arc of a circle oriented in a medio-lateral direction and, in the tibia plate (3), of a housing (34, 7, 10, 10', 54) designed to collaborate with the guide element belonging to the metal base (2) so as to allow the tibia plate (3) to slide in rotation about the centre of rotation (C, C') of the said upstand or peg.

2. Knee prosthesis according to Claim 1, **characterized in that** the metal base (2) comprises an upstand (22) in the shape of an arc of a circle having a central part (23) secured to lateral edges (24, 25) which are not as tall as the said central part, while the tibia plate (3) comprises, on its lower face (30), a housing (34) in the shape of an arc of a circle.

3. Knee prosthesis according to Claim 2, **characterized in that** the metal base (2) has, opposite the upstand (22), a retaining peg (26) borne by a centre of rotation so as to engage with a cutout (35) formed in the tibia plate (3) to prevent the latter from lifting off the base (2) as the said plate slides in rotation about its centre of rotation.

4. Knee prosthesis according to Claim 3, **characterized in that** the retaining peg (26) consists of a cylindrical pin (27) integral with a head (28) which has a larger diameter than the said pin so that the said head engages with inclined faces made in the cutout (35).

5. Knee prosthesis according to Claim 2, **characterized in that** the metal base (2) has, opposite the upstand (22), a centring peg (29) borne by the centre of rotation (C, C') so as to engage with a blind hole (37) formed in the tibia plate (3) to guide the latter with respect to the base (2) as the said plate slides in rotation about its centre of rotation.

6. Knee prosthesis according to Claim 1, **characterized in that** the metal base (2) and the tibia plate (3) respectively comprise a cutout (4 and 38) through which the posterior cruciate ligament can pass.

7. Knee prosthesis according to Claim 1, **characterized in that** the metal base (2) has two upstands (5 and 6) in the shape of an arc of a circle curved in the same direction and centred about the same centre of rotation (C, C'), while the tibia plate (3) comprises housings (7 and 8) intended to receive the said upstands (5 and 6) respectively, so as to allow the said plate to slide in rotation about the centre of rotation (C, C').

8. Knee prosthesis according to Claim 7, **characterized in that** the upstand (6) is integral with a flange (60) which engages in a slot (80) in the housing (8) to prevent the tibia plate (3) from lifting off the metal base (2) as the said plate slides in rotation about the centre of rotation (C, C').

9. Knee prosthesis according to Claim 1, **characterized in that** the metal base (2) comprises two upstands (5' and 9) in the shape of an arc of a circle in opposite directions and centred about the same centre of rotation (C, C'), while the tibia plate (3) comprises an element (10) and a housing (12) which are intended to receive the said upstands (5' and 9) respectively to allow the said plate to slide in rotation about the centre of rotation (C, C').

10. Knee prosthesis according to Claim 9, **characterized in that** the upstand (5') is set out on the external periphery of the horizontal disc (20) of the metal base (2) so as to engage with a peripheral recess (10) in the tibia plate (3).

11. Knee prosthesis according to Claim 9, **characterized in that** the upstand (9) is offset from the centre of rotation (C, C') and comprises a flange (90) which snap-fastens into the housing (12) in the tibia plate (3) to, on the one hand, guide the plate (3) as it slides in rotation about its centre (C, C') and, on the other hand, retain the said plate so that it does not lift off the metal base (2).

12. Knee prosthesis according to Claim 1, **characterized in that** the metal base (2) comprises a peripheral upstand (13) in the shape of an arc of a circle integral with a flange (14) directed towards the tibia bone vertical axis (YY') and a housing (15) set out in the region of the centre of rotation (C, C'), while the tibia plate (3) has, on its external periphery, a recess (10') in which there is formed a horizontal slot (16) intended to receive the flange (14) of the said upstand (13) and, on its lower face (30), a stub (17) which engages with the housing (15).

13. Knee prosthesis according to Claim 1, **characterized in that** the metal base (2) comprises three peripheral upstands (50, 51, 52) extending vertically above the horizontal disc (20), while the tibia plate (3) has, on its external periphery, three recesses (53, 54, 55) intended to receive the said upstands (50, 51, 52) respectively to allow the said plate to be guided as it slides in rotation about the centre of rotation (C, C').

14. Knee prosthesis according to Claim 1, **characterized in that** metal base (2) comprises at least two pegs (18) set out in an arc of a circle and defining a centre of rotation (C, C'), while the tibia plate (3) comprises a housing (34) of the same radius of curvature formed in the tibia plate (3).

15. Knee prosthesis according to Claim 14, **characterized in that** the pegs (18) are set out in an arc of a circle about a centre of rotation (C, C'), while the tibia plate (3) has a housing (34) intended to receive the said pegs.

16. Knee prosthesis according to Claim 14, **characterized in that** the pegs (18) have a centre of rotation (C') which is offset from the tibia bone vertical axis (YY'), while the said peg is a certain distance away from its centre of rotation.

17. Knee prosthesis according to Claim 1, **characterized in that** the rotational travel between the tibia plate (3) and the metal base (2) is reduced to zero when the dimensions of the housing (34, 7, 10, 10', 54) are made so as to engage without clearance with the upstand (22, 5, 5', 13, 51).

18. Knee prosthesis according to Claim 1, **characterized in that** the short height of the guide means and of their anterior positioning on the metal base (2) allows the tibia plate (3) to be mounted on the said base via a strictly anterior approach, the said plate requiring upward clearances only by the height of the said guide means.
